(19) Europäisches Patentamt
European Patent Office
Office européen des brevets

(11) Veröffentlichungsnummer: **0 028 714**
**B1**

(12) # EUROPÄISCHE PATENTSCHRIFT

(45) Veröffentlichungstag der Patentschrift: **15.09.82**

(21) Anmeldenummer: **80106199.5**

(22) Anmeldetag: **11.10.80**

(51) Int. Cl.³: **C 07 C 47/575,** C 07 C 45/29, B 01 J 23/62, B 01 J 23/64, B 01 J 27/02

(54) **Verfahren zur Herstellung von 3-Phenoxy-benzaldehyden.**

(30) Priorität: **30.10.79 DE 2943805**

(43) Veröffentlichungstag der Anmeldung: **20.05.81 Patentblatt 81/20**

(45) Bekanntmachung des Hinweises auf die Patenterteilung: **15.09.82 Patentblatt 82/37**

(84) Benannte Vertragsstaaten: **BE CH DE FR GB IT LI NL**

(56) Entgegenhaltungen:
**DE-A-2 620 254**
**Patents Abstracts of Japan Band 2, Nr. 110, 13. September 1978**

(73) Patentinhaber: **BAYER AG, Zentralbereich Patente, Marken und Lizenzen, D-5090 Leverkusen 1, Bayerwerk (DE)**

(72) Erfinder: **Wedemeyer, Karlfried, Dr., Bilharzstrasse 7, D-5000 Köln 80 (DE)**
Erfinder: **Fiege, Helmut, Dr., Walter-Flex-Strasse 8, D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 028 714 B1

BUNDESDRUCKEREI BERLIN

Verfahren zur Herstellung von 3-Phenoxy-benzaldehyden

Die Erfindung betrifft ein Verfahren zur Herstellung von 3-Phenoxy-benzaldehyden durch katalytische Oxidation von 3-Phenoxy-benzylalkoholen mit Sauerstoff oder Sauerstoff enthaltenden Gasen.

Es ist bekannt (Neuere Methoden der präparativen organischen Chemie, Band 2, Seite 213, 214 [1960]), Benzylalkohol mit Sauerstoff in Gegenwart von $PtO_2$ zu Benzaldehyd zu oxidieren. Die Oxidation von Benzylalkohol, einem in Wasser nur wenig löslichen Alkohol, zu Benzaldehyd ist aber nur in einem Lösungsmittel möglich, in dem Alkohol, Aldehyd und entstehendes Reaktionswasser vollständig löslich sind. Sobald eine wäßrige Phase auftritt, agglomeriert der Katalysator und die Oxidation kommt zum Stillstand. Als bestes Lösungsmittel wird n-Heptan empfohlen, in dem aber nur mit Benzylalkohol-Konzentrationen von etwa 2% gearbeitet werden kann. Die Benzaldehyd-Ausbeute beträgt in diesem Lösungsmittel 78% der Theorie.

Wird die Oxidation von Benzylalkohol in wäßrig/alkalischem Medium in Gegenwart eines Platin-Kohle-Kontaktes durchgeführt, entsteht kein Benzaldehyd, sondern praktisch quantitativ Benzoesäure.

Es ist ferner bekannt, daß sich 2- und 4-Hydroxybenzylalkohole in wäßrig-alkalischem Medium selektiv an Platinmetall-Kontakten oxidieren lassen, insbesondere wenn gleichzeitig noch bestimmte Unedelmetalle wie Blei, Bismut oder Tellur zugesetzt werden (DE-AI-2 620 254, JP-PA-5 373 532). Hier liegen jedoch spezielle Voraussetzungen für eine Oxidation vor: So sind sowohl die Hydroxybenzylalkohole als auch die Hydroxybenzaldehyde als Phenolderivate sehr gut in wäßrigem Alkali unter Salzbildung löslich, durch Resonanzstabilisierung wird die Aldehydbildung stark gefördert und zudem geht der Hydroxybenzaldehyd keine Cannizzaro-Reaktion mehr ein (s. Organic Reactions, Vol. II, S. 103–105, Wiley [1944]).

Es wurde nun ein Verfahren zur Herstellung von 3-Phenoxy-benzaldehyden durch Oxidation von 3-Phenoxy-benzylalkoholen der Formel

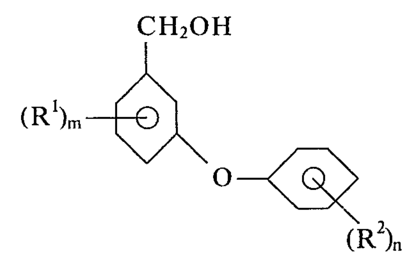

in der

m für 1 bis 4 steht,
n für 1 bis 5 steht,
$R^1$ und $R^2$ gleich oder verschieden sind und Wasserstoff, Halogen, Alkyl mit 1 bis 12 Kohlenstoffatomen, Cycloalkyl mit 3 bis 6 Kohlenstoffatomen, Aralkyl mit 7 bis 12 Kohlenstoffatomen, Alkoxy mit 1 bis 12 Kohlenstoffatomen, Cycloalkyl mit 5 bis 7 Kohlenstoffatomen, Phenoxy oder Perfluoralkyl mit 1 bis 4 Kohlenstoffatomen bedeuten,

in flüssiger Phasen gefunden, das dadurch gekennzeichnet ist, daß man die Oxidation mit Sauerstoff oder Sauerstoff enthaltenden Gasen in wäßrigem Alkali bei Temperaturen vom Erstarrungspunkt bis zum Siedepunkt des Reaktionsgemisches in Gegenwart von Platinmetall-Katalysatoren und in Gegenwart von Blei und/oder Bismut und/oder Tellur und/oder deren Verbindungen als Aktivatoren oder in Gegenwart von Palladium ohne Zusatz von Aktivatoren durchführt.

Als Alkylreste der Formel (I) kommen geradkettige oder verzweigte Kohlenwasserstoffe mit 1 bis 12, bevorzugt 1 bis 6, Kohlenstoffatomen in Frage.

Genannt seien: Methyl, Ethyl, Propyl, Isopropyl, Butyl, Isobutyl, sec.-Butyl, tert.-Butyl, Pentyl, Isopentyl, tert.-Amyl, Hexyl, Isohexyl, Heptyl, Isoheptyl, Octyl, Isooctyl, tert.-Octyl, Nonyl, Isononyl, Dodecyl und Isododecyl.

Bevorzugt genannt seien: Methyl, Ethyl, Propyl, Isopropyl, Butyl, Isobutyl, sek.-Butyl, tert.-Butyl, Pentyl, Isopentyl, tert.-Amyl und Isohexyl.

Als Cycloalkylreste kommen solche mit 3 bis 6, vorzugsweise 5 und 6, Kohlenstoffatomen in Betracht, wie der Cyclopentyl- und Cyclohexylrest.

Als Arylreste seien genannt: der Phenyl- und Naphthylrest, bevorzugt der Phenylrest.

Als Aralkylreste kommen solche mit 7 bis 12, bevorzugt 7 bis 9 Kohlenstoffatomen in Frage. Genannt seien: der Benzyl-, α-Methyl-benzyl-, α,α-Dimethyl-benzyl- und der α-Ethylbenzylrest, bevorzugt der Benzylrest.

Als Alkoxyreste kommen solche mit bis zu 12, bevorzugt bis zu 6, Kohlenstoffatomen in Betracht.

Zum Beispiel werden genannt: Methoxy, Ethoxy, Propoxy, Isopropoxy, Butoxy, Isobutoxy, tert.-Butoxy, Isopentoxy, Hexoxy, Isohexoxy und Methylendioxy.

Als Cycloalkoxyreste kommen solche mit bis zu 7, bevorzugt bis zu 6, Kohlenstoffatomen in Frage. Genannt seien bevorzugt der Cyclopentoxy- und der Cyclohexoxyrest.

Halogene können Fluor, Chlor, Brom, Jod, bevorzugt Fluor, Chlor und Brom, sein.

Als Perfluoralkylreste kommen solche mit bis zu 4, bevorzugt bis zu 2, Kohlenstoffatomen in Frage. Zum Beispiel werden genannt: der Trifluormethyl- und der Pentafluorethylrest, insbesondere der Trifluormethylrest.

Bevorzugt werden in das erfindungsgemäße Verfahren 3-Phenoxy-benzylalkohole der Formel

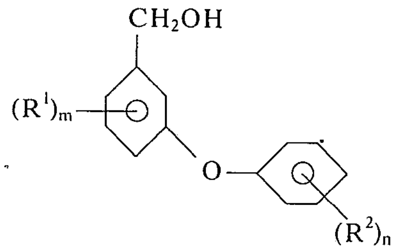

(II)

worin

m für 1 oder 2 steht,

n 1, 2 oder 3 bedeutet und

$R^1$ und $R^2$ gleich oder verschieden sind und für Wasserstoff, Alkyl mit 1 bis 6 Kohlenstoffatomen, Alkoxy mit 1 bis 6 Kohlenstoffatomen, Phenoxy, Fluor, Chlor, Brom und Trifluormethyl stehen,

eingesetzt.

Besonders bevorzugt werden in das erfindungsgemäße Verfahren 3-Phenoxy-benzylalkohol, 4-Fluor-3-phenoxybenzylalkohol, 6-Chlor-3-phenoxybenzylalkohol, 3-(4-Methylphenoxy)-benzylalkohol, 3-(4-Chlorphenoxy)-benzylalkohol, 3-(3,4-Dichlorphenoxy)-benzylalkohol und 3-[3-(Trifluormethyl)-phenoxy]-benzylalkohol eingesetzt.

Die Herstellung der 3-Phenoxy-benzylalkohole ist bekannt. Sie können hergestellt werden durch Hydrolyse von 3-Phenoxy-benzylchloriden oder 3-Phenoxy-benzylacetaten (DE-OS 2 402 457), durch Reduktion von 3-Phenoxy-benzoesäuren (US-PS 3 987 140) oder deren Ester (BE-PS 838 193), durch Reaktion von m-Hydroxy-benzylalkoholen mit Halogenbenzolen (JA-PS 4 861 443) oder durch Oxidation von 3-Phenoxy-toluolen (Agric. Biol. Chem. 37 [1973], 2682). Gegebenenfalls können bereits Rohgemische von 3-Phenoxy-benzylalkoholen aus solchen Herstellungsverfahren in die erfindungsgemäße Oxidation eingesetzt werden.

Als Alkali kommen Verbindungen der Alkali- und/oder der Erdalkalimetalle in Betracht, wie die Hydroxyde, Carbonate, Hydrogencarbonate, Phosphate und Borate. Besonders bevorzugt werden die Carbonate und/oder Hydroxyde des Natriums und/oder Kaliums als Alkali eingesetzt.

Die bei dem erfindungsgemäßen Verfahren einzusetzenden Mengen an Alkali können in weiten Grenzen schwanken. Im allgemeinen setzt man auf 1 Mol des zu oxidierenden 3-Phenoxy-benzylalkohols 0,01 bis 3 Mol, vorzugsweise 0,02 bis 1,5 Mol Alkali ein.

Das Alkali kann zu Beginn der Reaktion auf einmal in wasserfreier Form oder zusammen mit Wasser oder als wäßrige Lösung zum Reaktionsgemisch mit Wasser oder als wäßrige Lösung zum Reaktionsgemisch gegeben werden. Das Alkali kann aber auch in wasserfreier oder in in Wasser gelöster Form erst im Verlauf der Oxidation intermittierend oder kontinuierlich dem Reaktionsgemisch hinzugefügt werden.

Da bei der Oxidation der 3-Phenoxy-benzylalkohole zu den 3-Phenoxy-Benzaldehyden Wasser entsteht, läuft das Verfahren zwangsläufig auf eine Oxidation in Gegenwart von Wasser hinaus. Bemerkenswert ist bei dem erfindungsgemäßen Verfahren daß das Wasser die Oxidation auch dann nicht stört, wenn es eine zweite flüssige Phase bildet. Es kann sogar vorteilhaft sein, wenn man die wäßrige Phase durch Wasserzugabe noch vergrößert.

Das Volumenverhältnis von wäßriger Phase zu zu oxidierendem 3-Phenoxy-benzylalkohol (der organischen Phase) kann in weiten Grenzen schwanken. Zu Beginn der Reaktion kann das Verhältnis von wäßriger Phase zu organischer Phase 0 : 1 betragen. Dies ist der Fall, wenn die Oxidation unter Verwendung von wasserfreiem Alkali begonnen und auch kein Wasser zusätzlich hinzugefügt wird. Die Obergrenze des Volumenverhältnisses von wäßriger Phase zu organischer Phase wird man aus praktischen Gründen im allgemeinen nicht über 30 : 1 wählen. Bewährt haben sich insbesondere Volumenverhältnisse von etwa 0 : 1 bis etwa 15 : 1.

Im Reaktionsgemisch können während der Oxidation auch noch andere inerte, in Wasser schwer lösliche organische Verbindungen anwesend sein, die als Lösungsmittel für den 3-Phenoxy-benzylalkohol und/oder den entstehenden Aldehyd fungieren und zum Bestandteil der organischen Phase werden. Solche Lösungsmittel können aliphatische und/oder aromatische Kohlenwasserstoffe

und/oder aliphatische und/oder aromatische Ether sein. Genannt seien: Hexan, Heptan, Iso-octan, Benzol, Toluol, Xylol und 3-Phenoxy-toluol.

Als Platinmetalle werden in das erfindungsgemäße Verfahren Platin, Palladium, Rhodium, Iridium, Ruthenium und/oder Osmium eingesetzt. Bevorzugt werden die Platinmetalle Platin und/oder Palladium eingesetzt, insbesondere Platin.

Die als Katalysatoren eingesetzten Platinmetalle können den Reaktionskomponenten in verschiedenster Form zugegeben werden, beispielsweise in elementarer, d. h. metallischer Form, oder in Form von Verbindungen, z. B. als Oxide oder in Form anderer Verbindungen.

Die Platinmetalle können auch auf Träger aufgebracht sein. Als Träger geeignet sind beispielsweise Aktivkohle, Graphit, Kieselgur, Kieselgel, Spinelle, Aluminiumoxid, Asbest, Calciumcarbonat, Magnesiumcarbonat, Bariumsulfat oder auch organische Trägermaterialien. Bevorzugt wird als Trägermaterial Aktivkohle, beispielsweise aus Holz hergestellte billige Pulverkohlen, wie sie vielfach für Entfärbungszwecke verwendet werden, eingesetzt.

Der Platinmetallgehalt dieser Trägerkatalysatoren kann in weiten Grenzen schwanken. Im allgemeinen beträgt der Platinmetallgehalt 0,01 bis 20 Gew.-%, bevorzugt 0,05 bis 10 Gew.-%, besonders bevorzugt 0,1 bis 5 Gew.-%.

Die Mengen, in denen die Platinmetall-Katalysatoren — bezogen auf den 3-Phenoxy-benzylalkohol — verwendet werden, können in weiten Grenzen schwanken. Die Mengen hängen u. a. von der gewünschten Oxidationsgeschwindigkeit ab. Im allgemeinen ist ihre Menge geringer als die 3-Phenoxy-benzylalkoholmenge im Einsatzgemisch. Eingesetzt werden die Platinmetall-Katalysatoren üblicherweise in Mengen von 0,1 bis 30 Gew.-%, bevorzugt 0,5 bis 20 Gew.-%, bezogen auf den 3-Phenoxybenzylalkohol. Die Platinmetallkatalysatoren können im allgemeinen wiederholt für die Oxidation eingesetzt werden.

Die Aktivität der Platinmetallkatalysatoren wird durch die Gegenwart von Blei und/oder Bismut und/oder Tellur und/oder deren Verbindungen als Aktivatoren erheblich gesteigert. Bemerkenswert ist nicht nur diese Aktivitätssteigerung, sondern auch die Tatsache, daß die Oxidation trotz der Anwesenheit von Alkali nicht zu 3-Phenoxy-benzoesäure, sondern selektiv zum 3-Phenoxy-benzaldehyd führt.

Die Mengen, in denen die erfindungsgemäß zu verwendenden Aktivatoren eingesetzt werden, können in weiten Grenzen schwanken. Die Aktivatorwirkung macht sich bereits bei Zusätzen von $5 \times 10^{-6}$ Mol Metall oder Metallverbindung pro Mol 3-Phenoxy-benzylalkohol deutlich bemerkbar. Es können auch 0,1 Mol oder mehr Aktivator pro Mol 3-Phenoxy-benzylalkohol eingesetzt werden, jedoch bieten diese hohen Zusätze im allgemeinen keinen besonderen Vorteil. Üblicherweise werden die Aktivatoren in Mengen von $1 \times 10^{-5}$ bis $1 \times 10^{-1}$ Mol, vorzugsweise $2 \times 10^{-5}$ bis $2 \times 10^{-2}$ Mol, pro Mol zu oxidierendem 3-Phenoxy-benzylalkohol zugegeben.

Die erfindungsgemäß als Aktivatoren zu verwendenden Metalle können als solche, d. h. in elementarer Form und/oder in Form ihrer Verbindungen, z. B. als Oxyde, Hydroxyde, Oxydhydrate oder Sauerstoffsäuren oder als Salze von Wasserstoffsäuren, wie Chloride, Bromide, Jodide, Sulfide, Selenide, Telluride oder als Salze von anorganischen Sauerstoffsäuren, wie Nitrate, Nitrite, Phosphite, Phosphate, Carbonate, Sulfate, Sulfite, Perchlorate, Antimonate, Arseniate, Selenide, Seleniate, Tellurite, Tellurate, Borate, oder als Salze von Sauerstoffsäuren, die von Übergangsmetallen abstammen, wie Vanadate, Niobdate, Tantalate, Chromate, Molybdate, Wolframate, Permanganate, oder als Salze organischer aliphatischer oder aromatischer Säuren, wie Formiate, Acetate, Propionate, Benzoate, Salicylate, Lactate, Mandelate, Glyoxylate, Arylglyoxylate, Citrate oder als Phenolate eingesetzt werden.

Die Aktivatoren können im Reaktionsgemisch jeweils löslich, teilweise löslich oder unlöslich sein.

Die erfindungsgemäßen Aktivatoren können in unterschiedlichen und auch gemischten Wertigkeitsstufen vorliegen, auch können Änderungen in der Wertigkeit während der Reaktion eintreten. Sofern die Aktivatoren nicht bereits als Oxide und/oder Hydroxyde zugegeben werden, ist es möglich, daß sie sich im alkalischen Medium ganz oder teilweise in diese umwandeln. Nach der Reaktion kann der Platinmetall-Katalysator mit dem schwerlöslichen Aktivator abfiltriert und für weitere Oxidationsreaktionen verwendet werden. Verluste an Platinmetall-Katalysatoren und/oder Aktivatoren sind gegebenenfalls zu ersetzen.

Der Aktivator kann als Feststoff, vorzugsweise in feinverteilter Form, oder in gelöster Form den Reaktionskomponenten zugesetzt werden. Man kann den Aktivator auch schon bei der Herstellung des Platinmetall-Katalysators zugeben oder den Platinmetall-Katalysator mit dem Aktivator imprägnieren. Der Aktivator kann auch als Trägermaterial für das Platinmetall dienen.

Besonders bewährt hat sich die Kombination von Platin mit Blei und/oder Bismut und/oder Tellur.

Das erfindungsgemäße Verfahren kann bei Temperaturen vom Erstarrungspunkt bis zum Siedepunkt des Reaktionsgemisches durchgeführt werden. Dabei hängt der Erstarrungspunkt und der Siedepunkt des Reaktionsgemisches u. a. von dem Katalysatorsystem, der Alkalikonzentration, den Substanzeigenschaften der Einsatzprodukte und Endprodukte und dem angelegten Druck ab und ist leicht durch Vorversuche zu ermitteln.

Bevorzugt wird bei Temperaturen von 0 bis 110° C, besonders bevorzugt bei 20 bis 100° C, gearbeitet.

Die Reihenfolge, in der Platinmetall-Katalysator, der Aktivator, falls er nicht schon in dem

Platinmetall-Katalysator enthalten ist, das gegebenenfalls wäßrige Alkali, der 3-Phenoxy-benzylalkohol und das gegebenenfalls mitzuverwendende inerte organische Lösungsmittel zusammengegeben werden, ist beliebig. So kann man das gegebenenfalls wäßrige Alkali vorlegen, den Katalysator und den Aktivator zusetzen und dann den gegebenenfalls bereits in einem Lösungsmittel gelösten 3-Phenoxy-benzylalkohol zugeben. Man kann aber auch in umgekehrter Reihenfolge verfahren. Auch können der Platinmetall-Katalysator und der Aktivator dem Gemisch aus gegebenenfalls wäßrigem Alkali und dem (gegebenenfalls in einem Lösungsmittel gelösten) 3-Phenoxy-benzylalkohol hinzufügt werden. Beispielsweise ist es auch möglich, den Platinmetall-Katalysator, den Aktivator, einen Teil des gegebenenfalls wäßrigen Alkalis und den — gegebenenfalls in einem Lösungsmittel gelösten — 3-Phenoxy-benzylalkohol vorzulegen und den Rest des Alkalis erst im Verlauf der Reaktion einzudosieren. Von den zahlreichen Möglichkeiten sind hier nur wenige genannt. Während der Reaktion ist für eine gute Durchmischung der Komponenten zu sorgen.

Selbstverständlich ist es auch möglich, Gemische verschiedener 3-Phenoxy-benzylalkohole zu oxidieren.

Das erfindungsgemäße Verfahren kann üblicherweise so durchgeführt werden, daß man Sauerstoff oder Sauerstoff enthaltende Gase, wie Luft, mit dem Gemisch aus gegebenenfalls wäßrigem Alkali, Platinmetall-Katalysator, Aktivator, 3-Phenoxy-benzylalkohol und dem gegebenenfalls zugesetzten inerten Lösungsmittel in Kontakt bringt. Im allgemeinen führt man die Umsetzung bei Atmosphärendruck (1 bar) durch, jedoch kann auch bei höheren oder niedrigeren Drücken oxidiert werden. Im allgemeinen führt man das erfindungsgemäße Verfahren im Druckbereich von 0,5 bis 10 bar durch.

Der Verlauf der Oxidation kann über die aufgenommene Sauerstoffmenge verfolgt werden, und die Oxidation wird abgebrochen, wenn die für den gewünschten 3-Phenoxy-benzylalkohol-Umsatz erforderliche Sauerstoffmenge aufgenommen ist. Meist hört die Sauerstoffaufnahme in diesem Stadium von selbst auf oder sie verlangsamt sich. Der Fortgang der Reaktion kann auch auf andere Weise, z. B. durch Bestimmung des gebildeten 3-Phenoxy-benzaldehyds bestimmt werden. Es kann durchaus zweckmäßig sein, die Reaktion bei noch unvollständigem 3-Phenoxy-benzylalkohol-Umsatz abzubrechen.

Zur Aufarbeitung werden der Platinmetall-Katalysator und der ungelöste Aktivator abgetrennt, beispielsweise durch Filtration. Das erhaltene flüssige Reaktionsgemisch trennt sich im allgemeinen in eine wäßrige-alkalische Phase und in eine organische Phase auf, die den 3-Phenoxy-benzaldehyd enthält. Der 3-Phenoxy-benzaldehyd kann von der wäßrig-alkalischen Phase z. B. durch Dekantieren und/oder Extrahieren mit einem organischen, in Wasser schwer löslichen Lösungsmittel abgetrennt werden. Als Extraktionsmittel eignet sich besonders ein gegebenenfalls bereits zur Umsetzung zugesetztes inertes Lösungsmittel. Nach Trocknen des Extraktionsgemisches erhält man den 3-Phenoxy-benzaldehyd durch Destillation. Selbstverständlich ist es auch möglich, den Aldehyd auf andere Weise, z. B. über die entsprechende Alkali-Bisulfit-Additionsverbindung, zu isolieren.

Aus der wäßrig-alkalischen Phase kann die als Nebenprodukt entstandene 3-Phenoxy-benzoesäure durch Ansäuern freigesetzt und im allgemeinen durch Abfiltrieren isoliert werden. Man kann die noch alkalische wäßrige Phase aber auch bei einem der nächsten Ansätze ganz oder teilweise wiederverwenden.

Nach dem erfindungsgemäßen Verfahren können 3-Phenoxybenzaldehyde der Formel

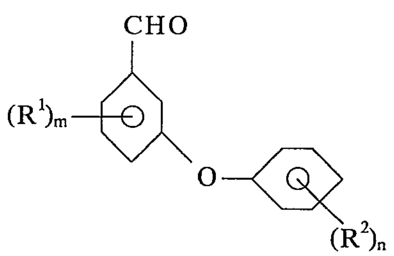

(III)

hergestellt werden, worin m, n und $R^1$ und $R^2$ die vorgenannte Bedeutung haben.

Nach dem erfindungsgemäßen Verfahren gelingt es, in einfacher Weise 3-Phenoxy-benzaldehyde im technischen Maßstab durch Oxidation von 3-Phenoxy-benzylalkoholen mit Sauerstoff oder Sauerstoff enthaltenden Gasen herzustellen. Dabei sind die gute Ausbeute und hohe Selektivität, mit der die Oxidationsreaktion vonstatten geht, besonders hervorzuheben.

Es ist völlig überraschend, daß im Gegensatz zu dem in der Literatur beschriebenen Oxidationsverfahren von Benzylalkohol, die erfindungsgemäße Oxidation in wäßrig alkalischem Medium durchgeführt werden kann, ohne daß Benzoesäurederivate in größeren Mengen gebildet werden.

Weiterhin überrascht es, daß die Aktivität der erfindungsgemäßen Platinmetall-Katalysatoren nicht beeinträchtigt wurde, obwohl man die Reaktion in wäßrigem Medium durchführte. Es war nämlich zu erwarten, daß der Katalysator durch das Reaktionswasser zusammengeballt würde, wodurch die Reaktion sofort zum Stillstand käme (vgl. Neuere Methoden der präparativen organischen Chemie,

Band 2, Seite 214, rechte Spalte [1960]).

Die nach dem erfindungsgemäßen Verfahren herstellbaren 3-Phenoxy-benzaldehyde sind wertvolle Zwischenprodukte, z. B. für die Synthese hochwirksamer Pyrethroid-Insektizide und anderer biologisch aktiver Wirkstoffe (s. Nachr. Chem. Techn. Lab. 26 [1978], S. 120—122).

Die nachfolgenden Beispiele sollen das erfindungsgemäße Verfahren verdeutlichen, ohne es jedoch auf diese Beispiele einzuschränken.

### Beispiel 1

In einem mit Rührer, Thermometer und Gaszuleitung versehenen, über einen Außenmantel thermostatisierbaren Reaktionsgefäß werden 0,5 g pulverförmige Aktivkohle mit einem Platingehalt von 1 Gew.-%, 100 ml 0,25 n-Natronlauge, 0,5 ml 0,5 m-$Pb(NO_3)_2$-Lösung (entsprechend einer Bleimenge von $2{,}5 \cdot 10^{-4}$ Mol) und 10,0 g 3-Phenoxy-benzylalkohol (Reinheit laut Gaschromatographie 98,8%) eingebracht.

Nach Verdrängen der Luft aus dem Reaktionsgefäß durch Sauerstoff wird der Rührer angestellt, das Reaktionsgemisch auf 80° C gebracht und bei dieser Temperatur unter kräftigem Rühren Sauerstoff unter Normaldruck in die Mischung eingeleitet. Nach 40 Minuten sind ca. 0,026 Mol $O_2$ aufgenommen, und die Sauerstoffaufnahme kommt nahezu zum Stillstand.

Nach Abfiltrieren des Kontaktes wird das aus einer den 3-Phenoxy-benzaldehyd enthaltenden organischen Phase und einer die 3-Phenoxy-benzoesäure enthaltenden wäßrig-alkalischen Phase bestehende Filtrat (Reaktionsgemisch) mit Ether extrahiert. Nach Trocknen der vereinigten Etherextrakte über Natriumsulfat und Abziehen des Ethers unter Stickstoff verbleiben 9,4 g Rückstand, der nach der gaschromatographischen Analyse 96,9% 3-Phenoxy-benzaldehyd und 1,3% noch nicht umgesetzten 3-Phenoxy-benzylalkohol enthält. Daraus ergibt sich ein 3-Phenoxy-benzylalkohol-Umsatz von 98,8%, eine 3-Phenoxy-benzaldehyd-Ausbeute von 93% der Theorie und eine 3-Phenoxy-benzaldehyd-Selektivität von 94%.

Aus der wäßrig-alkalischen Phase werden durch Ansäuern mit 20%iger Salzsäure auf pH 1, Abfiltrieren, Waschen mit etwas Wasser und Trocknen 0,6 g 3-Phenoxy-benzoesäure vom Fp. 146 bis 147° C erhalten, entsprechend 5,7% der Theorie, bezogen auf den eingesetzten 3-Phenoxy-benzylalkohol.

Der abfiltrierte Kontakt kann wiederverwendet werden.

### Beispiel 2

Es wird wie in Beispiel 1 gearbeitet, jedoch mit dem Unterschied, daß nicht Blei-, sondern $2{,}5 \cdot 10^{-4}$ Mol Bismut in Form seines feingepulverten Nitrats $Bi(NO_3)_3 \cdot 5\,H_2O$ als Aktivator zur Reaktionsmischung gegeben werden. Nach einer Oxidationszeit von 40 Minuten sind wieder 0,026 Mol $O_2$ aufgenommen, und die Oxidation kommt praktisch zum Stillstand. Die wie vorstehend durchgeführte Aufarbeitung ergibt 9,1 g 3-Phenoxy-benzaldehyd, der laut Gaschromatographie 97,5%ig und laut Oxim-Titration 97,8%ig ist, sowie 0,7 g 3-Phenoxy-benzoesäure vom Fp. 146 bis 147° C. Die Ausbeute an 3-Phenoxy-benzaldehyd beträgt mithin 91% der Theorie, die an 3-Phenoxy-benzoesäure 7% der Theorie.

### Vergleichsbeispiel 1

Es wird wie in Beispiel 1 gearbeitet, jedoch mit dem Unterschied, daß weder Blei noch Bismut zur Reaktionsmischung gegeben werden.

Man stellt fest, daß ohne Zusatz dieser Aktivatoren kaum Sauerstoff aufgenommen wird: Nach 40 Minuten sind erst 5% der sonst in dieser Zeit verbrauchten Sauerstoffmenge aufgenommen. Danach verlangsamt sich die Geschwindigkeit der $O_2$-Aufnahme noch mehr.

### Vergleichsbeispiel 2

Es wird wie in Beispiel 1 gearbeitet, jedoch mit dem Unterschied, daß anstelle der 100 ml Natronlauge nur 100 ml Wasser zum Reaktionsgemisch gegeben werden.

Man stellt fest, daß trotz der Anwesenheit von Blei in Abwesenheit von Alkali unter diesen Bedingungen kein Sauerstoff aufgenommen wird (Versuchsdauer über 2 Stunden).

Zusammen mit dem Beispiel 1 und dem Vergleichsbeispiel 1 zeigt dieser Versuch, daß die gleichzeitige Anwesenheit von Alkali und Aktivator erfindungswesentlich ist.

Beispiele 3 bis 23

Diese Beispiele wurden in der in Beispiel 1 beschriebenen Apparatur in prinzipiell gleicher Arbeitsweise durchgeführt. Die im Einzelfall gewählten Versuchsbedingungen sind der Tabelle 1 zu entnehmen. Für den als Ausgangsverbindung eingesetzten 3-Phenoxy-benzylalkohol wird in Tabelle 1 die Abkürzung 3-POB verwendet.

Wie die Ergebnisse in Tabelle 1 zeigen, kann das erfindungsgemäße Verfahren unter hinsichtlich Temperatur, Alkali/3-POB-Verhältnis, Volumenverhältnis von wäßriger zu organischer Phase, Anwesenheit von inerten organischen Lösungsmitteln, Gewichtsverhältnis von Katalysator zu 3-POB, Aktivatorart, Aktivatorform und Aktivatorkonzentration sehr verschiedenen Bedingungen bei hohen Umsätzen mit sehr guten 3-Phenoxy-benzaldehyd-Ausbeuten bzw. -Selektivitäten durchgeführt werden.

Tabelle 1

3-Phenoxybenzylalkohol (3-POB) unter verschiedenen Bedingungen

| Bei-spiel-Nr. | 3-POB-Einsatz | | Mol NaOH | wäßrige Phase[a]) | g Kataly-sator[b]) | Aktivator-Zusatz | | Temp. | $O_2$-Aufnahme | | 3-POB | 3-Phenoxy-benzaldehyd | | 3-Phenoxy-benzoe-säure |
|---|---|---|---|---|---|---|---|---|---|---|---|---|---|---|
| | g | % ig | Mol 3-POB | organ. Phase | g 3-POB | Art | Mol-Akti-vator / Mol 3-POB | °C | Mol $O_2$ / Mol 3-POB | in Minuten | Umsatz % | Aus-beute % d.Th. | Selek-tivität % | Ausbeute % d. Th. |
| 3 | 20 | 49,0[c]) | 2,04 | 5 : 1 | 0,1 | $Pb(NO_3)_2$ | $5 \cdot 10^{-3}$ | 30 | 0,47 | 30 | 94,0 | 84,6 | 90,0 | 4,3 |
| 4 | 10 | 98,8 | 1,01 | 10 : 1 | 0,05 | $Bi(NO_3)_3$ | $5 \cdot 10^{-3}$ | 60 | 0,51 | 35 | 97,6 | 91,6 | 93,9 | 5,6 |
| 5 | 10 | 98,8 | 0,06 | 10 : 1 | 0,05 | $Pb(NO_3)_2$ | $2,5 \cdot 10^{-3}$ | 98 | 0,51 | 150 | 99,0 | 93,4 | 94,4 | 4,7 |
| 6 | 10 | 98,8 | 0,02 | 10 : 1 | 0,05 | $Pb(NO_3)_2$ | $2,5 \cdot 10^{-3}$ | 90 | 0,36 | 60 | 73 | 69,1 | 94,7 | 1,0 |
| 7 | 10 | 98,8 | 0,506 | 10 : 1 | 0,05 | $Pb(NO_3)_2$ | $2 \cdot 10^{-2}$ | 80 | 0,53 | 45 | 99 | 90,6 | 91,5 | 7,5 |
| 8 | 10 | 98,8 | 0,506 | 10 : 1 | 0,05 | $Pb(NO_3)_2$ | $4 \cdot 10^{-5}$ | 80 | 0,48 | 45 | 93,2 | 89,1 | 95,6 | 3,8 |
| 9 | 10 | 98,8 | 0,506 | 10 : 1 | 0,05 | $Pb(NO_3)_2$ | $2 \cdot 10^{-5}$ | 80 | 0,36 | 60 | 69,0 | 66,3 | 96,1 | 2,4 |
| 10 | 10 | 97,9 | 0,143 | 10 : 1 | 0,05 | $Bi(NO_3)_3$ | $2 \cdot 10^{-2}$ | 80 | 0,51 | 45 | 97,7 | 90,3 | 92,4 | 5,2 |
| 11 | 10 | 97,9 | 0,143 | 10 : 1 | 0,05 | $Bi(NO_3)_3$ | $3 \cdot 10^{-4}$ | 80 | 0,53 | 45 | 99,5 | 91,9 | 92,4 | 6,7 |
| 12 | 10 | 97,9 | 0,143 | 10 : 1 | 0,05 | $Bi(NO_3)_3$ | $4 \cdot 10^{-5}$ | 80 | 0,50 | 45 | 95,2 | 88,2 | 93,3 | 5,2 |
| 13 | 20 | 97,9 | 0,255 | 5 : 1 | 0,025 | $Pb(NO_3)_2$ | $2,5 \cdot 10^{-3}$ | 80 | 0,52 | 80 | 98,0 | 91,5 | 93,4 | 5,7 |
| 14 | 40 | 97,9 | 0,128 | 2,5 : 1 | 0,0125 | $Pb(NO_3)_2$ | $1,3 \cdot 10^{-3}$ | 80 | 0,50 | 150 | 96,5 | 90,4 | 93,7 | 4,8 |
| 15 | 70 | 97,9 | 0,073 | 1,4 : 1 | 0,0071 | $Pb(NO_3)_2$ | $7,3 \cdot 10^{-4}$ | 80 | 0,51 | 400 | 99 | 90,1 | 91 | 5,3 |
| 16 | 20 | 97,9 | 0,051 | 0 : 1 | 0,05 | $Bi(NO_3)_3$ | $5 \cdot 10^{-3}$ | 80 | 0,30 | 45 | 58,2 | 56,0 | 96,2 | 3,3 |
| 17 | 10 | 98,8 | 0,506 | 10 : 1 | 0,05 | Pb-Pulver | $5 \cdot 10^{-3}$ | 80 | 0,52 | 50 | 98,0 | 90,8 | 92,7 | 6,1 |

Fortsetzung

| Beispiel-Nr. | 3-POB-Einsatz | | Mol NaOH | wäßrige Phase[a]) | g Katalysator[b]) | Aktivator-Zusatz | | Temp. | $O_2$-Aufnahme | | 3-POB | 3-Phenoxybenzaldehyd | | 3-Phenoxybenzoesäure |
|---|---|---|---|---|---|---|---|---|---|---|---|---|---|---|
| | g | % ig | Mol / 3-POB | organ. Phase | g 3-POB | Art | Mol-Aktivator / Mol 3-POB | °C | Mol $O_2$ / Mol 3-POB | in Minuten | Umsatz % | Ausbeute % d.Th. | Selektivität % | Ausbeute % d. Th. |
| 18 | 10 | 98,8 | 0,506 | 10 : 1 | 0,05 | Bi-Pulver | $5 \cdot 10^{-3}$ | 80 | 0,52 | 45 | 98,0 | 92,9 | 93,8 | 6,1 |
| 19 | 10 | 98,8 | 0,506 | 10 : 1 | 0,05 | Te-Pulver | $5 \cdot 10^{-3}$ | 80 | 0,5 | 250 | 99 | 93,9 | 94,8 | 3,3 |
| 20 | 10 | 98,8 | 0,506 | 10 : 1 | 0,05 | $TeO_2$ | $5 \cdot 10^{-3}$ | 80 | 0,50 | 200 | 95,4 | 90,5 | 94,9 | 4,7 |
| 21 | 10 | 98,8 | 0,506 | 10 : 1 | 0,05 | $H_6TeO_6$ | $5 \cdot 10^{-3}$ | 80 | 0,50 | 150 | 95 | 90,2 | 94,9 | 4,7 |
| 22 | 10 | 98,8 | 0,506 | 10 : 1 | 0,05 | $PbO_2$ | $5 \cdot 10^{-3}$ | 80 | 0,51 | 55 | 97,4 | 91,3 | 93,7 | 5,2 |
| 23 | 20 | 49,0[d]) | 0,506 | 5 : 1 | 0,05 | $Pb(OCOCH_3)_2$ | $5 \cdot 10^{-3}$ | 80 | 0,55 | 60 | ~100 | 86,8 | – | 11,4 |

[a]) Volumenverhältnis zu Beginn der Oxidation.
[b]) Katalysator = 1% Platin auf Aktivkohle.
[c]) Rest vorwiegend 3-Phenoxytoluol als Lösungsmittel.
[d]) Rest vorwiegend Toluol als Lösungsmittel.

Beispiele 24 bis 29

Die Oxidation verschiedenartig substituierter 3-Phenoxy-benzylalkohole mit Sauerstoff von 1 bar bei 80° C in Gegenwart von 100 ml 0,25 n-Natronlauge, 0,5 g pulverförmiger Aktivkohle mit 1 Gew.-% Platingehalt und $1{,}25 \cdot 10^{-4}$ Mol Blei(II)-nitrat führte zu den in Tabelle 2 zusammengestellten Ergebnissen. Apparatur und Durchführung der Versuche entsprachen dem Beispiel 1.

Tabelle 2

Oxidation von substituierten 3-Phenoxy-benzylalkoholen zu den entsprechend substituierten 3-Phenoxy-benzaldehyden

| Beispiel Nr. | Eingesetzter substituierter 3-Phenoxy-benzylalkohol | | | $O_2$-Aufnahme | | Alkohol-Umsatz | substituierter 3-Phenoxy-benzaldehyd | | substit. 3-Phenoxy-benzoesäure |
|---|---|---|---|---|---|---|---|---|---|
| | Bezeichnung | Einsatz g | Reinheit % | mol $O_2$ / mol 3-POB | in min | % | Ausbeute % d. Th. | Selektivität % | Ausbeute % d. Th. |
| 24 | 4-Fluor-3-phenoxy-benzylalkohol | 10,9 | 96 | 0,51 | 20 | >99 | 95 | – | 4,5 |
| 25 | 6-Chlor-3-phenoxy-benzylalkohol | 11,7 | 93 | 0,33 | 40 | 56 | 46,2 | 82,5 | 9,5 |
| 26 | 3-(4-Methylphenoxy)-benzylalkohol | 10,7 | 97 | 0,51 | 30 | 98 | 94,3 | 96,2 | 3,6 |
| 27 | 3-(4-Chlor-phenoxy)-benzyl-alkohol | 11,7 | 95 | 0,52 | 30 | >99 | 94,5 | – | 4,4 |
| 28 | 3-(3,4-Dichlor-phenoxy)-benzyl-alkohol | 13,5 | 94 | 0,50 | 30 | 97 | 94,3 | 97,2 | 1,5 |
| 29 | 3-[3-(Trifluor-methyl)-phenoxy]-benzylalkohol | 13,3 | 95 | 0,50 | 25 | >99 | 97,2 | – | 0,8 |

Beispiele 30 bis 32

In der in Beispiel 1 beschriebenen Apparatur und Arbeitsweise wird in Gemische aus jeweils 10 g 3-Phenoxy-benzylalkohol, 100 ml 0,1 n-Natronlauge und 0,5 g pulverförmiger Medizinalkohle mit 5 Gew.-% Palladiumgehalt bei 80° C Sauerstoff unter Normaldruck eingerührt. Die Gemische enthalten im Falle des Beispiels 30 noch $2{,}5 \cdot 10^{-4}$ Mol Wismut(III)-nitrat, im Falle des Beispiels 31 noch $2{,}5 \cdot 10^{-4}$ Mol Tellursäure ($H_6TeO_6$) und im Falle des Beispiels 32 keinen weiteren Aktivatorzusatz.

Die in Tabelle 3 zusammengestellten Ergebnisse zeigen, daß an Palladium bereits ohne Aktivatorzusatz oxidiert werden kann, daß der Zusatz von Wismut oder Tellur jedoch zu einer Verbesserung von Aktivität und Ausbeute bzw. Selektivität führt:

Tabelle 3

Oxidation an Palladium

| Beispiel Nr. | Aktivator | Zeit*) min | 3-POB-Umsatz % | 3-Phenoxy-benzaldehyd Ausbeute % d. Th. | 3-Phenoxy-benzaldehyd Selektivität % | 3-Phenoxy-benzoesäure % d. Th. |
|---|---|---|---|---|---|---|
| 30 | $Bi(NO_3)_3$ | 90 | 90 | 75 | 83 | 12 |
| 31 | $H_6TeO_6$ | 150 | 98 | 79 | 81 | 15 |
| 32 | ohne | 250 | 83 | 61 | 73 | 17 |

*) Für die Aufnahme von ca. 0,5 Mol $O_2$ pro Mol 3-POB.

**Patentansprüche**

1. Verfahren zur Herstellung von 3-Phenoxy-benzaldehyden durch Oxidation von 3-Phenoxy-benzylalkoholen der Formel

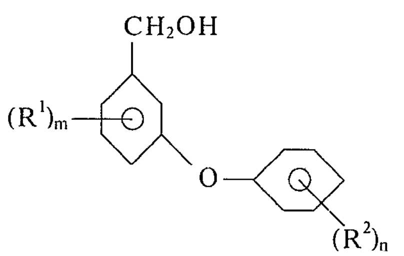

in der

m für 1 bis 4 steht,
n für 1 bis 5 steht,
$R^1$ und $R^2$ gleich oder verschieden sind und Wasserstoff, Halogen, Alkyl mit 1 bis 12 Kohlenstoffatomen, Cycloalkyl mit 3 bis 6 Kohlenstoffatomen, Aralkyl mit 7 bis 12 Kohlenstoffatomen, Alkoxy mit 1 bis 12 Kohlenstoffatomen, Cycloalkoxy mit 5 bis 7 Kohlenstoffatomen, Phenoxy oder Perfluoralkyl mit 1 bis 4 Kohlenstoffatomen bedeuten,

in flüssiger Phase, dadurch gekennzeichnet, daß man die Oxidation mit Sauerstoff oder Sauerstoff enthaltenden Gasen in wäßrigem Alkali bei Temperaturen vom Erstarrungspunkt bis zum Siedepunkt des Reaktionsgemisches in Gegenwart von Platinmetall-Katalysatoren und in Gegenwart von Blei und/oder Bismut und/oder Tellur und/oder deren Verbindungen als Aktivatoren oder in Gegenwart von Palladium ohne Zusatz von Aktivatoren durchführt.

2. Verfahren nach Anspruch 1, dadurch gekennzeichnet, daß man als Platinmetall-Katalysatoren Platin und/oder Palladium einsetzt.

3. Verfahren nach Anspruch 1, dadurch gekennzeichnet, daß das Platinmetall auf einen Träger aufgebracht ist.

4. Verfahren nach Ansprüchen 1 und 3, dadurch gekennzeichnet, daß man als Träger für das Platinmetall Aktivkohle verwendet.

5. Verfahren nach Ansprüchen 1, 3 und 4, dadurch gekennzeichnet, daß der Platinmetallgehalt der Trägerkatalysatoren 0,01 bis 20 Gew.-% beträgt.

6. Verfahren nach Anspruch 1, dadurch gekennzeichnet, daß man Blei und/oder Bismut und/oder Tellur und/oder deren Verbindungen als Aktivatoren in Mengen von $1 \cdot 10^{-5}$ bis $1 \cdot 10^{-1}$ Mol pro Mol zu oxidierendem 3-Phenoxy-benzylalkohol einsetzt.

7. Verfahren nach Ansprüchen 1 bis 6, dadurch gekennzeichnet, daß man pro Mol zu oxydierendem 3-Phenoxy-benzylalkohol 0,01 bis 3 Mol Alkali einsetzt.

8. Verfahren nach Ansprüchen 1 bis 7, dadurch gekennzeichnet, daß man als Alkali die Hydroxyde und/oder Carbonate des Natriums und/oder Kaliums einsetzt.

**Claims**

1. Process for the preparation of 3-phenoxy-benzaldehydes by oxidation of 3-phenoxy-benzyl alcohols of the formula

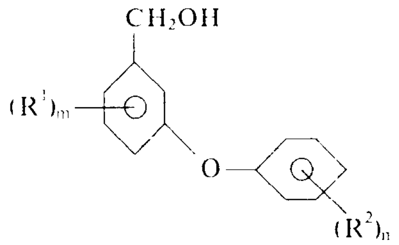

in which

m represents a number from 1 to 4,
n represents a number from 1 to 5 and
$R^1$ and $R^2$ are identical or different and denote hydrogen, halogen, alkyl with 1 to 12 carbon atoms, cycloalkyl with 3 to 6 carbon atoms, aralkyl with 7 to 12 carbon atoms, alkoxy with 1 to 12 carbon atoms, cycloalkoxy with 5 to 7 carbon atoms, phenoxy or perfluoroalkyl with 1 to 4 carbon atoms,

in a liquid phase, characterised in that the oxidation is carried out with oxygen or gases containing oxygen, in aqueous alkali, at temperatures from the solidification point to the boiling point of the reaction mixture, in the presence of platinum metal catalysts and in the presence of lead and/or bismuth and/or tellurium and/or compounds thereof as activators or in the presence of palladium without the addition of activators.

2. Process according to Claim 1, characterised in that platinum and/or palladium are employed as the platinum metal catalysts.

3. Process according to Claim 1, characterised in that the platinum metal is applied to a support.

4. Process according to Claims 1 and 3, characterised in that acitve charcoal is used as the support for the platinum metal.

5. Process according to Claims 1, 3 and 4, characterised in that the platinum metal content of the supported catalyst is 0.01 to 20% by weight.

6. Process according to Claim 1, characterised in that lead and/or bismuth and/or tellurium and/or compounds thereof are employed as activators in amounts of $1 \times 10^{-5}$ to $1 \times 10^{-1}$ mol per mol of 3-phenoxy-benzyl alcohol to be oxidised.

7. Process according to Claims 1 to 6, characterised in that 0.01 to 3 mols of alkali are employed per mol of 3-phenoxy-benzyl alcohol to be oxidised.

8. Process according to Claims 1 to 7, characterised in that the hydroxides and/or carbonates of sodium and/or potassium are employed as the alkali.

**Revendications**

1. Procédé de production de 3-phénoxy-benzaldéhydes par oxydation d'alcools 3-phénoxy-benzyliques de formule:

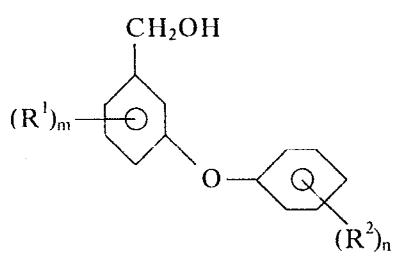

dans laquelle:

m a une valeur de 1 à 4,
n a une valeur de 1 à 5,
$R^1$ et $R^2$ sont égaux ou différents et représentent l'hydrogène, un halogène, un reste alkyle ayant 1 à 12 atomes de carbone, un reste cycloalkyle ayant 3 à 6 atomes de carbone, un reste aralkyle ayant 7 à 12 atomes de carbone, un reste alkoxy ayant 1 à 12 atomes de carbone, un reste cycloalkoxy ayant 5 à 7 atomes de carbone, un reste phénoxy ou perfluoralkyle ayant 1 à 4 atomes de carbone,

en phase liquide, caractérisé en ce qu'on conduit l'oxydation avec de l'oxygène ou des gaz contenant de l'oxygène dans un alcali aqueux à des températures allant du point de solidification au point d'ébullition du mélange réactionnel en présence de catalyseurs métalliques du type platine et en présence, comme activateurs, de plomb et/ou de bismuth et/ou de tellure et/ou de leurs composés ou en présence de palladium sans addition d'activateurs.

2. Procédé suivant la revendication 1, caractérisé en ce qu'on utilise le platine et/ou le palladium comme catalyseurs métalliques du type platine.

3. Procédé suivant la revendication 1, caractérisé en ce que le métal du type platine est déposé sur un support.

4. Procédé suivant les revendications 1 à 3, caractérisé en ce qu'on utilise du charbon actif comme support pour le métal du type platine.

5. Procédé suivant les revendications 1, 3 et 4, caractérisé en ce que la teneur en métal du type platine des catalyseurs fixés sur un support va de 0,01 à 20% en poids.

6. Procédé suivant la revendication 1, caractérisé en ce qu'on utilise le plomb et/ou le bismuth et/ou le tellure et/ou leurs composés comme activateurs en quantités de $1 \cdot 10^{-5}$ à $1 \cdot 10^{-1}$ moles par mole d'alcool 3-phénoxy-benzylique à oxyder.

7. Procédé suivant les revendications 1 à 6, caractérisé en ce qu'on utilise 0,01 à 3 moles d'alcali par mole d'alcool 3-phénoxy-benzylique à oxyder.

8. Procédé suivant les revendications 1 à 7, caractérisé en ce qu'on utilise comme alcali les hydroxydes et/ou carbonates de sodium et/ou de potassium.